# EUROPEAN PATENT SPECIFICATION

(11) **EP 4 324 858 B1**
(45) Date of publication and mention of the grant of the patent: **09.12.2026**
(21) Application number: 22787895.6
(22) Date of filing: 07.03.2022
(51) Int. Cl.: C08F 8/14, C07K 14/165, C07K 16/08, C07K 16/10, C08F 220/54, C08F 220/56, C08F 220/60, G01N 33/53, G01N 33/569

(54) **COPOLYMER, ANTIBODY-COPOLYMER CONJUGATE PREPARATION KIT, ANTIBODY-COPOLYMER CONJUGATE, ANTIGEN CONCENTRATION METHOD, AND ANTIGEN DETECTION METHOD**
COPOLYMER, KIT ZUR HERSTELLUNG EINES ANTIKÖRPER-COPOLYMER-KONJUGATS, ANTIKÖRPER-COPOLYMER-KONJUGAT, ANTIGENKONZENTRATIONSVERFAHREN UND ANTIGENERKENNUNGSVERFAHREN
COPOLYMÈRE, KIT DE PRÉPARATION DE CONJUGUÉ ANTICORPS-COPOLYMÈRE, CONJUGUÉ ANTICORPS-COPOLYMÈRE, PROCÉDÉ DE CONCENTRATION D'ANTIGÈNE ET PROCÉDÉ DE DÉTECTION D'ANTIGÈNE

(30) Priority: 12.04.2021 JP 2021066984
(43) Date of publication of application: 21.02.2024
(73) Proprietor: National Institute for Materials Science, Tsukuba-shi, Ibaraki 305-0047 (JP)
(72) Inventor: EBARA, Mitsuhiro, Tsukuba-shi, Ibaraki 305-0047 (JP); TOLBA, Ahmed Nabil Ahmed Elsayed, Tsukuba-shi, Ibaraki 305-0047 (JP)
(74) Representative: J A Kemp LLP
(86) International application number: PCT/JP2022/009632
(87) International publication number: WO 2022/219967

(56) References cited:
- WO-A1-2016/068271
- US-A1- 2017 304 420
- US-A1- 2021 000 934
- AHMED NABIL: "Temperature responsive smart polymer for enabling affinity enrichment of current coronavirus (SARS-CoV-2) to improve its diagnostic sensitivity", vol. 19, 1 January 2021 (2021-01-01), Sweden, pages 3609 - 3617, XP093154144, ISSN: 2001-0370, Retrieved from the Internet <URL:https://www.sciencedirect.com/science/article/pii/S2001037021002543/pdfft?md5=ff7324d9a0023028f6c32bc2509190ff&pid=1-s2.0-S2001037021002543-main.pdf> DOI: 10.1016/j.csbj.2021.06.016
- JACQUES SIMON ET AL: "Polymer/DNA Bio-conjugates via Grafting-from and Grafting-to", 1 November 2017 (2017-11-01), XP093299613, Retrieved from the Internet <URL:https://flex.flinders.edu.au/file/30ce6ada-a329-4964-af5b-4f06afcd6dd8/1/Thesis%20Simon%20JMC%20Bou%20%28Polymer-DNA%20Bio-conjugates%20via%20Grafting-from%20and%20Grafting-to%20strategies%29%20submitted%2017%20July%202017%20approved%2020.11.17.pdf#:~:text=details)%20were%20functionalised%20with%20a>
- NOOR FAIZAH CHE HARUN ET AL: "Artificial Control of Gene Silencing Activity Based on siRNA Conjugation with Polymeric Molecule Having Coil-Globule Transition Behavior", BIOCONJUGATE CHEMISTRY - AUTHOR MANUSCRIPT, vol. 27, no. 9, 18 August 2016 (2016-08-18), US, pages 1961 - 1964, XP055534705, ISSN: 1043-1802, DOI: 10.1021/acs.bioconjchem.6b00322
- MAEDA T ET AL: "Importance of bound water in hydration-dehydration behavior of hydroxylated poly(N-isopropylacrylamide)", JOURNAL OF COLLOID AND INTERFACE SCIENCE, ACADEMIC PRESS,INC, US, vol. 302, no. 2, 15 October 2006 (2006-10-15), pages 467 - 474, XP024909827, ISSN: 0021-9797, [retrieved on 20061015], DOI: 10.1016/J.JCIS.2006.06.047

## Description

### Technical Field

The present invention relates to an antibody-copolymer conjugate, a method for concentrating an antigen, and a method for detecting an antigen.

### Background Art

"Temperature-responsive polymers" that cause phase transition in response to a temperature change in the solution and change the physical properties are known. As such a temperature-responsive polymer, PTL 1 describes a polymer of "an isopropylacrylamide derivative including an azido group or an alkyne group.

### Citation List

### Patent Literature

PTL 1: Japanese Unexamined Patent Application Publication No. 2012-201634
PTL 2: US 2021/0000934 A1
PTL 2 relates to an adjuvant comprising Pattern Recognition Receptor (PRR) agonist molecules linked to polymer chains that are capable of undergoing particle formation in aqueous conditions, or in aqueous conditions in response to external stimuli; and methods of treatment or prevention of disease using such an adjuvant.

### Summary of Invention

### Technical Problem

In diagnosis of COVID-19 (SARS-CoV-2), a high false-positive rate is a problem due to the low amount of virus, antibody, and/or antigen (especially antigen) in the specimen, and there is a demand for a method for easily concentrating a test target object and improving the test sensitivity.

Accordingly, described herein is a copolymer that can be used for concentration of an antigen by immobilizing an antibody. Also described herein is an antibody-copolymer conjugate preparation kit. An object of the present invention is to provide an antibody-copolymer conjugate, a method for concentrating an antigen, and a method for detecting an antigen.

### Solution to Problem

The present inventors have intensively studied to solve the above problems and, as a result, found that the above objects can be achieved by the following formations.
[1] An antibody-copolymer conjugate including a unit 1 described later and a unit 7 described later.
[2] The antibody-copolymer conjugate according to [1], wherein Ab in the antibody-copolymer conjugate is an anti-SARS-CoV-2 antibody.
[3] A method for concentrating an antigen, including preparing a mixture solution A containing the antibody-copolymer conjugate according to [1] or [2] and an antigen corresponding to the antibody to form a complex of the antibody-copolymer conjugate and the antigen, and heating the mixture solution A to 20°C to 40°C to precipitate the complex.
[4] The method for concentrating an antigen according to [3], further including binding a linker represented by the formula 6 described later and the antibody via an amide bond derived from an amino group of the antibody to prepare an antibody-linker complex, preparing a mixture solution B containing the antibody-linker complex and a copolymer including the unit 1 described later and a unit 4 described later, and reacting and binding an azido group of the antibody-linker complex and an alkynylene group of the copolymer to each other in the mixture solution B to generate the antibody-copolymer conjugate.
[5] The method for concentrating an antigen according to [3] or [4], wherein the antibody is an anti-SARS-CoV-2 antibody.
[6] The method for concentrating an antigen according to [5], wherein the antigen is a SARS-CoV-2 protein.
[7] The method for concentrating an antigen according to [4], wherein a ratio of the content of the copolymer to the content of the antibody contained in the antibody-linker complex in the mixture solution B is 0.5 to 30 on a molar basis.
[8] A method for detecting an antigen, including concentrating an antigen by the method for concentrating an antigen according to any one of [3] to [7], and detecting the concentrated antigen.

### Advantageous Effects of Invention

As described herein, a copolymer that can be used for concentrating an antigen can be provided by immobilizing an antibody. Also described herein is an antibody copolymer conjugation preparation kit. According to the present invention, an antibody-copolymer conjugate, a method for concentrating an antigen, and a method for detecting an antigen can be provided.

### Brief Description of Drawings

[Fig. 1] Fig. 1 shows synthesized HIPAAm visualized by thin-layer chromatography (TLC).
[Fig. 2] Fig. 2 shows the results of ¹H NMR analysis of an HIPAAm monomer.
[Fig. 3] Fig. 3 shows the results of ¹H NMR analysis of P(NIPAAm-co-HIPAAm).
[Fig. 4] Fig. 4 shows the results of ¹H NMR analysis of specific copolymer (I).
[Fig. 5] Fig. 5 shows the measurement results of lower critical solution temperature (LCST) of synthesized P(NIPAAm-co-HIPAAm).
[Fig. 6] Fig. 6 shows the measurement results of LCST of specific copolymer (I).
[Fig. 7] Fig. 7 shows the measurement results of LCST of a conjugate of an antibody and specific copolymer (I).
[Fig. 8] Fig. 8 shows the results of measurement of the introduction amount of azido-(EG)₄-NHS per COVID-19 antibody with fluorescamine.
[Fig. 9] Fig. 9 shows the results of evaluation of click reaction of an antibody and a synthetic polymer at various concentrations by SDS-PAGE.
[Fig. 10] Fig. 10 is a graph showing the concentration rates of a COVID-19 recombinant protein when the concentration of antibody conjugate is changed.
[Fig. 11] Fig. 11 shows evaluation of the concentration effect of a COVID-19 antibody-temperature responsive polymer by lateral flow immunoassay (LFIA).
[Fig. 12] Fig. 12 shows evaluation of the concentration effect of a COVID-19 antibody-temperature responsive polymer by lateral flow immunoassay (LFIA).
[Fig. 13] Fig. 13 shows the measurement results of LCST of synthesized copolymer (II).
[Fig. 14] Fig. 14 shows the results of measurement of the introduction amount of azido-(EG)₄-NHS per COVID-19 antibody (S protein-capturing antibody) with fluorescamine.

### Description of Embodiments

The present invention will now be described in detail.

Although the explanation of the constituent elements described below may be made based on representative embodiments of the present invention, the present invention is not limited to the embodiments. For the avoidance of doubt, the present invention is defined by the scope of the claims.

In the present specification, a numerical range represented by using "to" means a range including the numerical values described before and after "to" as lower and upper limits.

### [Copolymer]

The copolymer (hereinafter, also referred to as "specific copolymer") described herein is a copolymer including a repeating unit (unit 1) represented by the following formula 1 and a repeating unit (unit 4) represented by the following formula 4.

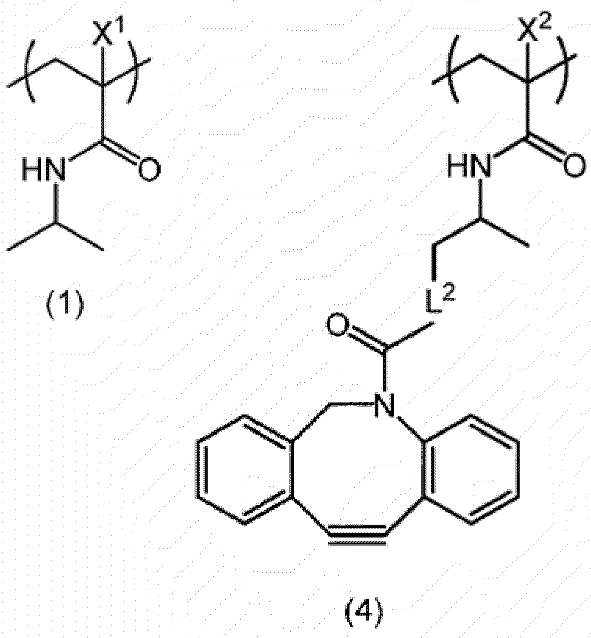

### (Unit 1)

A polymer constituted of the unit 1 only shows temperature responsiveness of a lower critical solution temperature (LCST) of 32°C in water. The specific copolymer has temperature responsiveness by including the unit 1.

The content of the unit 1 included in the specific copolymer is not particularly limited and is typically preferably 1 to 99 mol% when the total of repeating units is defined as 100 mol%. In particular, from the viewpoint of more sensitive temperature responsiveness of the specific copolymer and/or from the viewpoint of easily controlling the LCST within a temperature range suitable for concentration of an antigen (specifically, easily controlling the LCST to room temperature to about 40°C), the content of the unit 1 in the specific copolymer is preferably greater than 50 mol%, more preferably 60 mol% or more and preferably 97 mol% or less, more preferably 90 mol% or less.

In the formula 1, X¹ represents a hydrogen atom or a linear or branched alkyl group having 1 to 6 carbon atoms and is, from the viewpoint of more sensitive temperature responsiveness of the specific copolymer, preferably a hydrogen atom or a linear alkyl group having 1 to 4 carbon atoms and more preferably a hydrogen atom or a methyl group.

The unit 1 is preferably, but not particularly limited to, a unit based on a monomer represented by the following formula 1':

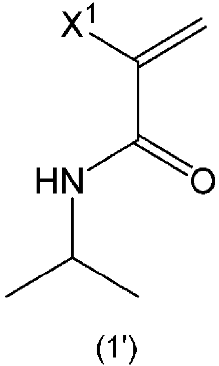

In the formula 1', X¹ is synonymous with X¹ in the formula 1, and the suitable forms thereof are the same. The monomer represented by the formula 1' may be synthesized by, for example, a known method such as the method described in Examples described later or may be a commercially available product.

### (Unit 4)

The specific copolymer includes a unit 4 represented by the formula 4. The unit 4 has one site (click reaction site) including cyclic alkyne (alkynylene group) per repeating unit and can easily form a complex by binding to an azido group of a linker described later through a click reaction. For example, a protein can be immobilized to the specific copolymer via a linker by binding the linker to the protein (such as antibody) in advance. That is, an antibody-copolymer conjugate can be produced.

The produced conjugate aggregates and causes precipitation at a temperature of the LCST or higher due to the temperature responsiveness derived from the specific copolymer. By using this, an antigen corresponding to the antibody can be concentrated.

The content of the unit 4 in the specific copolymer is not particularly limited and is typically preferably 1 to 99 mol% when the total of repeating units of the specific copolymer is defined as 100 mol%.

In particular, from the viewpoint of lowering the LCST and more sensitively detecting the antigen after concentration, the content is preferably 1 mol% or more, more preferably 2 mol% or more, further preferably 5 mol% or more and preferably 30 mol% or less, more preferably 20 mol% or less.

The unit 4 is typically more hydrophobic compared to other repeating units, and the LCST can be reduced by increasing the content of the unit 4.

In addition, the higher the content of the unit 4, the easier the introduction of an antibody. At the same time, in consideration of use for concentration by heating to 20°C to 40°C, the content of the unit 4 is preferably 1 mol% or more, more preferably 2 mol% or more, further preferably 5 mol% or more, particularly preferably 10 mol% and preferably 30 mol% or less, more preferably 20 mol% or less.

In the formula 4, X² represents a hydrogen atom or a linear or branched alkyl group having 1 to 6 carbon atoms. In the formula 4, L² represents a divalent group,. L² is not particularly limited and is preferably at least one selected from the group consisting of -O-, -S-, -C(O)-, -C(O)O-, -OC(O)O-, -NR^{A}- (R^{A} is a hydrogen atom or a monovalent substituent), a linear, branched, or cyclic aliphatic hydrocarbon group having 1 to 20 carbon atoms, a monocyclic or condensed ring aromatic hydrocarbon group having 6 to 20 carbon atoms, and a combination thereof.

In particular, from the viewpoint of obtaining a copolymer having more excellent effects of the present invention, more preferred is one group selected from the group consisting of -O-, -C(O)-, -NR^{A}-, a linear alkylene group having 1 to 10 carbon atoms, and a combination thereof, further preferred is at least one group selected from the group consisting of -O-, -C(O)-, and -NR^{A}-, and particularly preferred is -0-, -C(O)-, or -NH-.
The method for synthesizing the unit 4 is not particularly limited, and although a known synthesis method can be used, a method for obtaining the unit 4 by binding a precursor compound to a unit 4' represented by the following formula 4' is preferable in that the unit 4 can be obtained more easily.

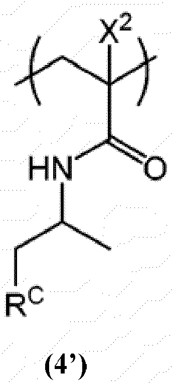

In the formula 4', R^{C} is a reactive substituent, and examples thereof include a hydroxy group, an amino group, a carboxy group, a glycidyl group, a mercapto group, a hydroxysuccinimide ester, and maleimide, and preferred is a hydroxy group.

The precursor compound is a compound including a click reaction site and a group that can react with the R^{C}, and a commercially available product may be used, or a product synthesized by a known method may be used. In particular, when a precursor compound including a carboxy group is used, the specific copolymer can be synthesized more easily.

As the method for synthesizing a precursor compound, for example, the method described in the scheme 1 of Chemical Communications (2010), 46(1), 97-99 can be used.

As the precursor compound, a compound obtained by attaching a substituent such as an amino group, a hydroxy group, or a carboxy group to the click reaction site and a compound esterified with maleimide or hydroxysuccinimide, which are commercially available, can also be used.

In particular, the unit 4' is preferably a unit 5 represented by the following formula 5 in that the unit 4 can be obtained more easily. In this case, a precursor compound including a carboxy group may be typically used.

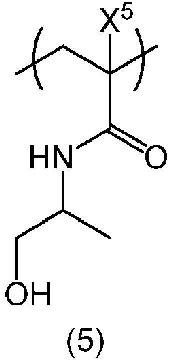

In synthesis of the specific copolymer, if unreacted unit 5 is present, in other words, when the specific copolymer includes the unit 1, the unit 4, and the unit 5, the LCST can be adjusted to higher temperature. Since the unit 5 is more hydrophilic compared to other repeating units, the LCST can be adjusted to higher temperature by increasing the content of the unit 5.

In the formula 5, X⁵ represents a hydrogen atom or a linear or branched alkyl group having 1 to 6 carbon atoms and is, from the viewpoint of more sensitive temperature responsiveness of the copolymer, preferably a hydrogen atom or a linear alkyl group having 1 to 4 carbon atoms and more preferably a hydrogen atom or a methyl group.

The content of the unit 5 in the specific copolymer is not particularly limited and is preferably 0 to 15 mol% when the total of repeating units of the specific copolymer is defined as 100 mol%.

The molecular weight of the specific copolymer is not particularly limited and is typically, as a number average molecular weight, preferably 2000 to 100000, more preferably 5000 to 50000, and further preferably 10000 to 30000.

When the number average molecular weight is 5000 or more, more excellent concentration efficiency is likely to be obtained (in other words, more excellent temperature responsiveness is likely to be obtained). At the same time, when the number average molecular weight is 50000 or less, a conjugate with an antibody is likely to be formed, or an antigen-antibody reaction is likely to proceed.

In particular, when an antibody-copolymer conjugate produced by the obtained copolymer is used, to obtain more excellent concentration efficiency, the number average molecular weight is preferably 15000 or more and more preferably 20000 or more. In general, many antibodies are highly hydrophilic, and in such a case, a copolymer having more excellent concentration efficiency is demanded. When the number average molecular weight of the copolymer is within the above numerical range, an excellent concentration efficiency is likely to be obtained.

The ratio of the molecular weight of the specific copolymer to the molecular weight of the antibody (specific copolymer/antibody) is not particularly limited but is preferably 1/30 to 1/5.

The molecular weight dispersity index (PDI) of the specific copolymer is not particularly limited and is, from the viewpoint of obtaining a copolymer having more excellent effects of the present invention, preferably 1.5 or less and more preferably 1.3 or less.

### (Other units)

The specific copolymer may include a repeating unit other than the above. Examples of the repeating unit other than the above include repeating units based on, for example, N-cyclopropylacrylamide (LCST = 46°C), N-n-propylacrylamide (LCST = 22°C), N-tetrahydrofurfurylacrylamide (LCST = 28°C), N-ethoxyethylacrylamide (LCST = 35°C), N-methyl-N-ethylacrylamide (LCST = 56°C), N-methyl-N-isopropylacrylamide (LCST = 23°C), N-methyl-N-n-propylacrylamide (LCST = 20°C), N,N-diethylacrylamide (LCST = 32°C), N-cyclopropylmethacrylamide (LCST = 59°C), N-isopropylmethacrylamide (LCST = 44°C), N-n-propylmethacrylamide (LCST = 28°C), and N-tetrahydrofurfurylmethacrylamide (LCST = 35°C).

### [Method for manufacturing specific copolymer]

The method for manufacturing the specific copolymer is not particularly limited and, from the viewpoint of being able to manufacture the specific copolymer more easily, preferably includes the following step (1) and the step (2) in this order.

Step (1): a step of copolymerizing monomers represented by the following formula 1' and formula 3' to obtain a copolymer (precursor).

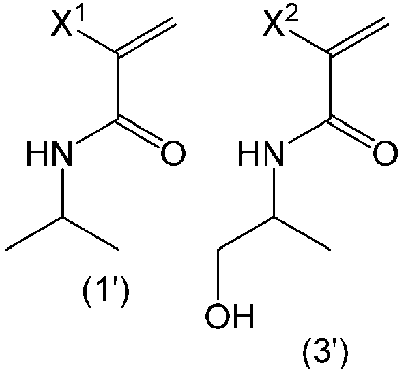

In the formulae 1' and 3', X¹ and X² are synonymous with X¹ in the formula 1 and X² in the formula 4', respectively, and the suitable forms thereof are also the same.

The method for copolymerizing the monomers is not particularly limited, and it is preferable to use living polymerization such as living radical polymerization, living anionic polymerization, and living cationic polymerization. In particular, from the viewpoint of obtaining a copolymer (precursor) more easily, living radical polymerization is preferable.

The living radical polymerization is based on that equilibrium between a small amount of propagating radical (free radical) species and a large amount of dormant species is rapidly established in a growth reaction by application of heat, light, a metal catalyst, or the like. Various types of living radical polymerization by dormant chains have been proposed.

For example, there are an ATRP method (atom transfer radical polymerization) using halogenated alkyl as a dormant species, a RAFT method (reversible addition fragmentation chain transfer) using thioester as a dormant species, and an NMP method (nitroxide mediated polymerization) using alkoxyamine as a dormant species.

The RAFT method is a method for polymerizing a vinyl-based monomer by adding a chain transfer agent having a high chain-transfer constant called a RAFT agent to a system of usual radical polymerization. As the RAFT agent, thioester can be used.

The amount of the RAFT agent can be appropriately selected depending on the molecular weight of the target copolymer. That is, since the RAFT agent binds to the terminal of each copolymer, for example, when the target is a 100-mer copolymer, the RAFT agent may be used at an amount of 0.1 to 3 mol% with respect to 100 mol% of the monomer.

The radical polymerization initiator to be used for the RAFT polymerization is not particularly limited and may be appropriately selected from known initiators such as an azo compound, a peroxide, and a redox-type initiator.

Examples of the azo compound include 2,2'-azobisisobutyronitrile (AIBN), 2,2'-azobis-2,4-dimethylvaleronitrile, 2,2'-azobis(2-methylpropionamidine) dihydrochloride, and 4,4'-azobis(4-cyanovaleric acid).

The amount of the polymerization initiator is generally preferably 0.1 to 50 mol% with respect to 1 mol of the RAFT agent. The reaction of the RAFT method is determined by the radical polymerization initiator to be used, and is generally performed at 40°C to 150°C in many cases. Polymerization is performed under atmospheric pressure in many cases, but can also be performed under pressure.

The RAFT method can be performed in the absence of a solvent, but can also be performed in the presence of a solvent. The solvent that is used as necessary is not particularly limited, and a known solvent can be used. The reaction can also be performed in water, and the reaction also proceeds in emulsification polymerization. The emulsifier that is used at that time may be a nonionic emulsifier, a cationic emulsifier, or an anionic emulsifier that can be used in general emulsification polymerization.

Step (2): a step of obtaining a specific copolymer by reacting the obtained copolymer (precursor) and a precursor compound represented by a formula 10.

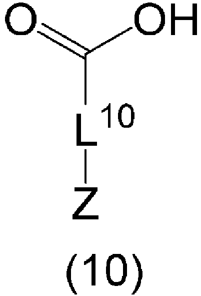

In the formula 10, Z is the group including the click reaction site (cyclic alkyne).

L¹⁰ represents a divalent group and is synonymous with L² in the formula 4, and the suitable forms thereof are also the same.

The desired specific copolymer is synthesized by forming an ester bond (condensation) between a hydroxy group of the copolymer (precursor) and a carboxy group of the precursor compound represented by the formula 10.

The method for forming an ester bond is not particularly limited, and examples thereof include a method by condensation reaction of a copolymer (precursor) and a precursor compound in the presence of a condensing agent, a catalyst, and a solvent at 0°C to 150°C (preferably 0°C to 100°C) for from 30 minutes to 24 hours (preferably 3 to 15 hours).

As the condensing agent, for example, triphenyl phosphite, N,N'-dicyclohexylcarbodiimide, 1-ethyl-3-(3-dimethylaminopropyl)carbodiimide hydrochloride, N,N'-carbonyldiimidazole, dimethoxy-1,3,5-triazinylmethyl morpholinium, O-(benzotriazol-1-yl)-N,N,N',N'-tetramethyluronium tetrafluoroborate, O-(benzotriazol-1-yl)-N,N,N',N'-tetramethyluronium hexafluorophosphate, and diphenyl (2,3-dihydro-2-thioxo-3-benzoxazolyl)phosphonate can be used. In particular, N,N'-dicyclohexylcarbodiimide is preferable. In this case, N,N-dimethyl-4-aminopyridine or the like can be used as a catalyst, and dichloromethane or the like can be used as a solvent.

### (Application of specific copolymer)

The specific copolymer has an LCST and a click reaction site and therefore specifically and irreversibly binds to, for example, a compound having an azido group dispersed or dissolved in a liquid medium such as water to form a complex. The complex rapidly aggregates and precipitates by heating this solution to a temperature of the LCST or higher and can be easily separated from the liquid medium.

For example, an antigen can be collected as a conjugate by forming a complex of a compound having an azido group and an antibody in advance and then producing it into a conjugate with a specific copolymer or mixing a compound having an azido group, an antibody, and a specific copolymer to produce a conjugate, and binding an antigen corresponding to the antibody for conjugation, and then heating the conjugate to the LCST or higher.

### [Antibody-copolymer conjugate]

The antibody-copolymer conjugate according to an embodiment of the present invention is an antibody-copolymer conjugate in which the copolymer already explained, and an antibody are bound to each other with a linker represented by the following formula 6.

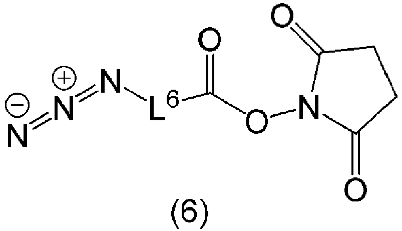

In the formula 6, L⁶ represents a divalent hydrocarbon group that may include a hetero atom, and is preferably a (poly)oxyalkylene group (the number of carbon atoms of the alkylene group is preferably 1 to 6) or a linear, branched, or cyclic hydrocarbon group having 1 to 20 carbon atoms, more preferably a polyoxyalkylene group.

The repeating number of the polyoxyalkylene group is preferably 2 to 10 and more preferably 2 to 8.

The linker represented by the formula 6 includes a N-hydroxysuccinimidyl ester (NHS ester) and can form an amide bond with a primary amine (for example, lysine residue) of a protein, i.e., binds to -NH₂ of an antibody to form a complex. At the same time, the linker includes an azido group and binds to an alkynylene group (click reaction site) of a specific copolymer to form a triazole ring.

The antibody-copolymer conjugate according to the present invention includes a repeating unit represented by the following formula 1 and a repeating unit represented by the following formula 7:

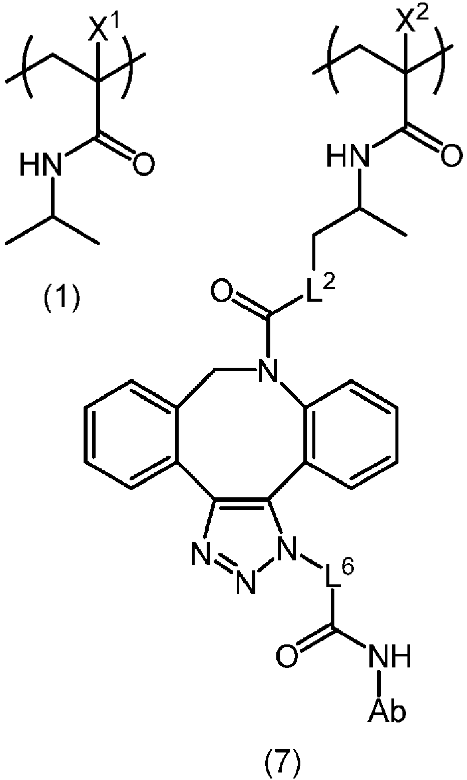

In the formula 7, X² represents a hydrogen atom or a linear or branched alkyl group having 1 to 6 carbon atoms and is synonymous with X² in the formula 4, and the suitable forms thereof are the same.

L² represents a divalent group and is synonymous with L² in the formula 4, and the suitable forms thereof are also the same. L⁶ represents a divalent hydrocarbon group that may include a hetero atom and is synonymous with L⁶ in the formula 6, and the suitable forms thereof are the same.

Ab represents an antibody residue. That is, it represents a state in which an amide bond is formed by a primary amine of the antibody and the NHS ester of the linker to immobilize the antibody to the specific copolymer via the linker.

In the state above, the antibody and the unit 7 are bound to each other at a 1 : 1 ratio, but the antibody-copolymer conjugate is not limited to the above state, and a state in which multiple primary amines of an antibody bind to alkynylene groups of a specific copolymer, respectively, via a linker, i.e., a product such as a crosslinked structure centered on the antibody molecule, may be formed.

The antibody-copolymer conjugate may include, in addition to the repeating units above, a unit 4 represented by the formula 4. That is, the conjugate may include an unreacted unit 4. As one embodiment, since the molecule of the specific copolymer is small compared to the molecule of the antibody, a large number of an antibody may be difficult to bind to a specific copolymer. In this case, unreacted unit 4 may remain on the antibody-copolymer conjugate.

The ratio of the antibody and the specific copolymer is not particularly limited, and as one embodiment, the amount of the copolymer is preferably 0.1 to 50 moles with respect to 1 mole of the antibody.

The antibody-copolymer conjugate may include another unit (unit 5) or the like that may be included in the specific copolymer, and the preferred range of the content of each unit is the same as the preferred range in the specific copolymer.

The antibody is not particularly limited, and a known antibody can be used. In particular, for example, preferred is an anti-SARS-CoV-2 antibody such as an anti-SARS-CoV-2 (COVID19) nucleocapsid (protein) antibody, and more preferred is an anti-SARS-CoV-2 spike (protein) antibody. In this case, in the antibody-copolymer conjugate, the content ratio of the content of the specific copolymer to the content of the antibody on a molar basis is not particularly limited, but is preferably 0.5 to 30.

### [Method for concentrating antigen]

The method for concentrating an antigen according to an embodiment of the present invention includes preparing a mixture solution A containing the antibody-specific copolymer conjugate and an antigen, forming a complex of the antibody-specific copolymer conjugate and the antigen, and heating the mixture solution A to 20°C to 40°C to precipitate the complex.

The mixture solution A can be prepared by adding the antibody-specific copolymer conjugate to, for example, a specimen obtained by diluting or dispersing saliva, sputum, or the like collected from a subject with or in a liquid medium (e.g., phosphate buffered saline) as necessary.

As long as the mixture solution A includes the antibody-specific copolymer conjugate and the antigen, an antigen may be added to an antibody-copolymer conjugate prepared in advance, an antigen may also be added to a solution of a mixture of a specific copolymer, a linker, and an antibody, or a specific copolymer, a linker, an antibody, and an antigen may also be mixed at one time.

Although the amount of the antibody-specific copolymer conjugate to be added to a specimen is not particularly limited, since there is a correlation between the addition amount and the concentration rate, the amount of the specific copolymer to be added is, for example, preferably 0.1 mg or more, more preferably 0.5 mg or more, and further preferably 1.0 mg or more, per 1 mg of the antigen protein in a specimen. The upper limit is not particularly limited and is generally preferably 5.0 mg or less.

More specifically, the amount of the specific copolymer to be added is preferably 0.1 to 10.0 mg for 1 mL of a specimen solution (e.g., including saliva).

On this occasion, the mixture solution A may further contain the specific copolymer in a free state (not forming a conjugate with an antibody). The addition of the specific copolymer makes the response to heat more sensitive and can concentrate an antigen more efficiently. The amount of the specific copolymer to be added is not particularly limited, but is preferably 0.1 to 20 times the content of the copolymer contained in the antibody-copolymer conjugate on a molar basis.

Furthermore, the method for concentrating an antigen preferably includes binding a linker already explained and an antibody via an amide bond derived from an amino group of the antibody to prepare an antibody-linker complex, preparing a mixture solution B containing the antibody-linker complex and a specific copolymer, and reacting and binding an azido group of the antibody-linker complex and an alkynylene group of the copolymer in the mixture solution B to generate an antibody-specific copolymer conjugate.

The antigen that can be concentrated by this method is not particularly limited and can be appropriately selected according to the type of the antibody of the antibody-copolymer conjugate. In particular, the antigen is preferably a SARS-CoV-2 protein and more preferably, for example, a SARS-CoV-2 spike protein or a SARS-CoV-2 nucleocapsid protein.

### [Kit]

An antibody-specific copolymer conjugate preparation kit as described herein includes a copolymer already explained and a linker. The kit may further include a solvent and so on as long as a specific copolymer and a linker are included.

The specific copolymer and the linker may be previously added to a developing solution of an antigen test kit by immunochromatography. In this case, the specific copolymer and the linker may be in a state of being previously bound to each other in the developing solution (solution).

### [Antigen test kit]

The antigen test kit as described herein is an antigen test kit including a specific copolymer already explained and a linker. This antigen test kit may include a known antigen test device in addition to the above.

The specific configuration of the antigen test device is not particularly limited, but is preferably a device that is used in a test by an immunological method. For example, the device may be a test piece that is generally used in a test by immunochromatography or may be a device having another configuration.

The specific configuration of the antigen test kit is not particularly limited. For example, the specific copolymer, the linker, and the antigen test device may be in a separated state. Alternatively, the specific copolymer, the linker, and the antigen test device may be in an integrated state.

### EXAMPLES

The present invention will now be described by Examples but is not limited thereto.

### [Synthesis of HIPAAm]

HIPAAm (hydroxy isopropyl acrylamide) was synthesized by the following procedure. D,L-2-Amino-1-propanol (0.15 mol) and triethylamine (0.15 mol) were well dissolved in anhydrous chloroform and were stirred at 5°C for 20 minutes, and acryloyl chloride (0.15 mol) was then slowly added thereto, followed by stirring at 5°C for 2 hours.

After evaporation of the solvent, redissolving in 2-propanol was performed, and the solution was left at -20°C for 24 hours or more. Finally, salts were removed by filtration, followed by concentration and purification by column chromatography. Synthesis of HIPAAm was verified by thin-layer chromatography (TLC) and ¹H NMR (nuclear magnetic resonance) (solvent was D₂O).

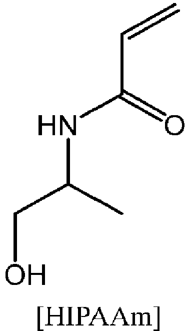

### [Synthesis of P(NIPAAm-co-HIPAAm)]

P(NIPAAm-co-HIPAAm) was synthesized by RAFT polymerization of HIPAAm and NIPAAm as shown by the following scheme. A solution including NIPAAm (1.89 g), HIPAAm (0.11 g), AIBN (1.31 mg), CDT (cyanomethyl dodecyl trithiocarbonate, 12.7 mg), and ethanol (17.6 mL) was stirred at 20°C for 20 hours, followed by evaporation and vacuum drying.

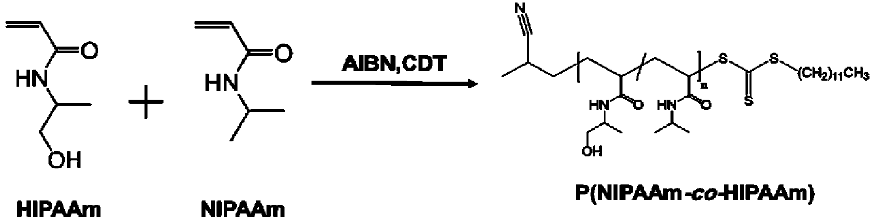

### [Synthesis of P(NIPAAm-co-HIPAAm-co-SAKIPAAm)]

As in the following scheme, dibenzylcyclooctynoic acid (DBCO) having an alkynylene group was introduced into the hydroxy group of HIPAAm by dehydration condensation to obtain a clickable responsive polymer P(NIPAAm-co-HIPAAm-co-SAKIPAAm).

A solution including DCM (dichloromethane, 30 mL), DBCO acid (35.8 mg), DMAP (4-dimethylaminopyridine, 12 mg), P(NIPAAm-co-HIPAAm) (100 mg), and DCC (N',N'-dicyclohexylcarbodiimide, 20 mg) was stirred overnight, followed by evaporation and vacuum drying. Hereinafter, the obtained P(NIPAAm-co-HIPAAm-co-SAKIPAAm) is also referred to as "copolymer (I)".

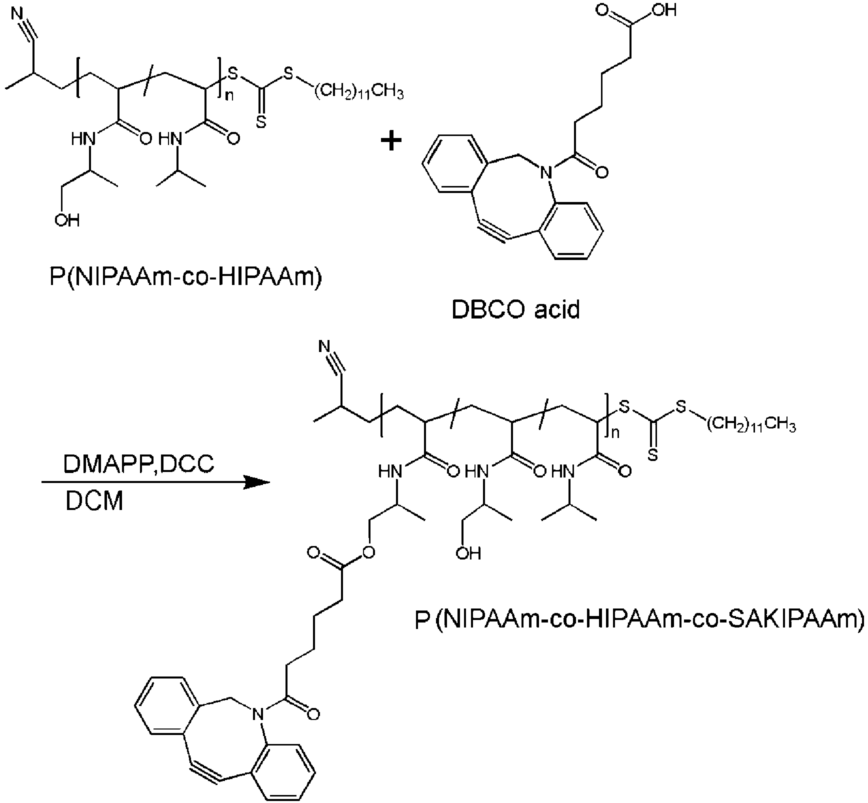

### [Evaluation of polymer synthesis]

The structure and the molecular weight of each of P(NIPAAm-co-HIPAAm) and copolymer (I) were verified by ¹H NMR (solvent: D₂O, DMSO) and gel permeation chromatography (GPC) (solvent: DMF (N,N-dimethylformamide), reference material: polystyrene), respectively. The lower critical solution temperature (LCST) was verified using a phosphate buffered saline (PBS) (pH: 7.4, concentration: 2.0 mg/mL, temperature: 10°C to 40°C) as a solvent.

### [Introduction of azido group to antibody]

An azido-PEG4-NHS ester (azido-ethylene glycol (EG4)-NHS ester, Tokyo Chemical Industry Co., Ltd.) powder was dissolved in DMSO (10 mg/mL), and azido-PEG4-NHS ester was prepared at different concentrations using a carbonate buffer (PH 8.6). The azido-PEG4-NHS ester was added to an antibody (anti-COVID-19 monoclonal antibody, > 95%, manufactured by MyBioSource, Inc., anti-COVID-19 antibody :: anti-viral COVID 19 nucleocapsid (NP) humanized coronavirus monoclonal antibody) at different supply ratios, followed by stirring at 4°C for 5 hours to introduce the azido group to the antibody as shown in the following formula:

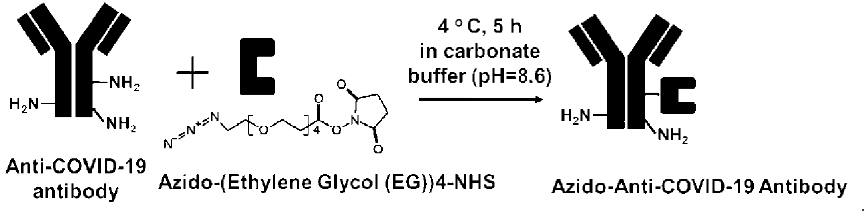

After introduction of the azido group to the antibody, the fluorescence of the antibody sample was measured using "Infinite" 200 PRO plate reader as follows. The azido-modified antibody sample was diluted to 0.5 mg/mL and was applied to a 96-microwell plate containing DL-2-aminobutyric acid (50 µL/well, reference material) at different concentrations, and 5 µL of fluorescamine (50 mg/mL) was then added to each well, followed by incubation in a dark place for 15 minutes. The fluorescence was then measured (395 nm/495 nm).

### [Production of COVID-19 antibody-temperature responsive copolymer conjugate by click reaction]

An azido-modified antibody was conjugated to an alkynylene group-modified temperature responsive copolymer using click chemistry as follows. A mixture of 75 µL of the copolymer (I) and 50 µL of an azido-modified anti- COVID-19 antibody (2.5 mg/mL in PBS) was stirred at 4°C for 12 hours as shown in the following formula.

The concentration of the copolymer (I) was adjusted such that the ratio of the antibody and the copolymer (I) was 1 : 1, 1 : 2, 1 : 4, 1 : 8, 1 : 15, or 1 : 30 (each of them is on a molar basis). The characteristics of the successfully obtained conjugate were revealed by sodium dodecylsulfate polyacrylamide gel electrophoresis (SDS-PAGE) compared to the cases of using the antibody and the copolymer (I) individually.

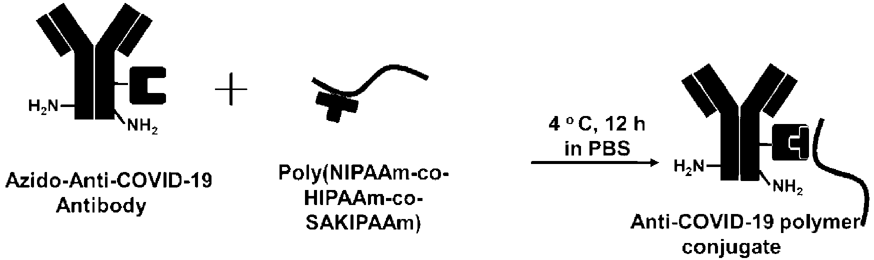

### [Evaluation of enrichment effect of COVID-19 antibody-temperature responsive copolymer]

The enrichment effect of the COVID-19 antibody-copolymer conjugate (1 : 1) prepared above was evaluated by a thermal precipitation method. The purified COVID-19-copolymer conjugate (100 µL of PBS solution with a concentration of 1.3 mg/mL includes 15 equivalents of the specific copolymer in a free state) was added in a microtube and was centrifuged (13000 × g) at 37°C for 5 minutes. Subsequently, the supernatant (90 µL) and the precipitate (10 µL) were collected, and the absorbance at 280 nm was measured with an UV visible absorption spectrometer. The ratios on a molar basis of the COVID-19 antibody in the liquid phase and in the solid phase were estimated, and the concentration ratios were calculated.

### [Evaluation of effect of antigen enrichment by COVID-19 antibody-temperature responsive copolymer]

The purified COVID-19 antibody-copolymer conjugate (1.3, 0.7, 0.35, 0.17, 0.08, and 0 mg/mL, 50 µL PBS, including 15 equivalents of copolymer (I) unit) was evaluated for the ability of concentrating a COVID-19 recombinant protein (manufactured by MyBioSource, Inc.).

The COVID-19 antibody-copolymer conjugate (at different concentrations, 50 µL PBS) was mixed with a COVID-19 recombinant protein (1.0 mg/mL, 50 µL PBS), followed by incubation for 1 hour. A free polymer was then added to the solution, followed by centrifugation in a microtube at 37°C, at 13000 × g, for 5 minutes.

The supernatant (90 µL) and the precipitate (10 µL) were collected, and the COVID-19 recombinant protein in the supernatant was measured using a modified lowery protein assay kit according to the indication of the maker. Finally, the enrichment ratios were calculated.

### [Evaluation of antigen concentration effect of COVID-19 antibody-temperature responsive copolymer using lateral flow immunoassay strip]

The COVID-19 recombinant protein was tested at different concentrations (50 to 1000 pg/mL, 20 µL PBS) using a lateral flow immunoassay strip. The COVID-19 recombinant protein (50 and 100 pg/mL, 100 µL PBS) was mixed with the COVID-19 antibody-specific copolymer conjugate (1.3 mg/mL, 100 µL PBS), followed by incubation for 1 hour. Subsequently, 15 equivalents of a free polymer [copolymer (I)] was added thereto, followed by centrifugation in a microtube at 37°C, at 13000 × g, for 5 minutes.

The supernatant (180 µL) and the precipitate (20 µL) were collected, and concentrated portion was measured by LFIA.

### [Result]

### (Confirmation of HIPAAm synthesis)

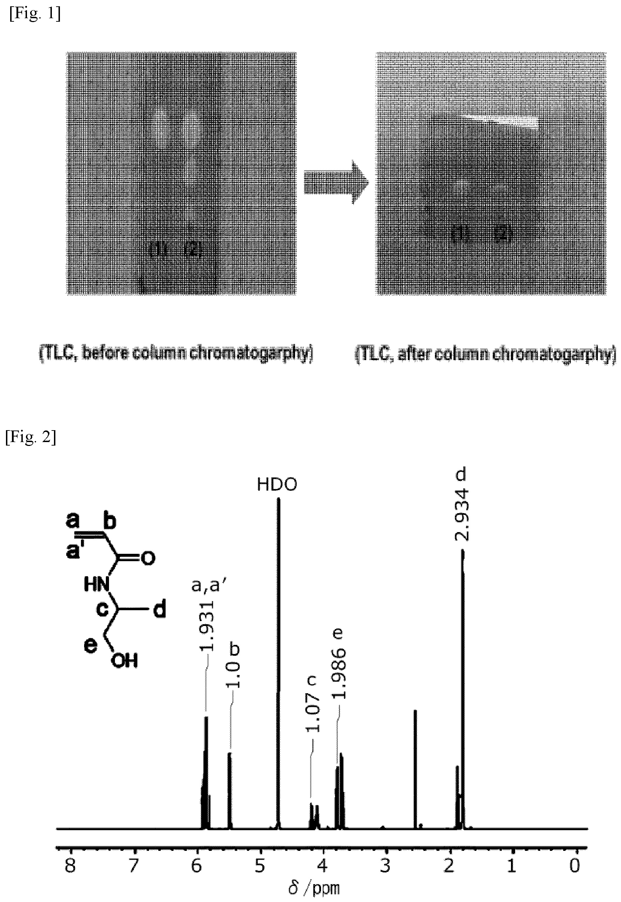

The purification of an HIPAAm monomer was confirmed by TLC analysis. Fig. 1 shows the images of an HIPAAm standard sample "(1)" and a synthesized sample "(2)" before and after column purification (solid phase: silica gel plate, mobile phase: ethyl acetate, visualizing agent: KMnO₄ solution).

According to Fig. 1, HIPAAm was successfully prepared together with two main secondary products. As shown in Fig. 1, HIPAAm was purified by column chromatography, and finally the synthesis of HIPAAm was confirmed using the results of ¹H NMR (Fig. 2).

### (Confirmation and characterization of P(NIPAAm-co-HIPAAm) and copolymer (I))

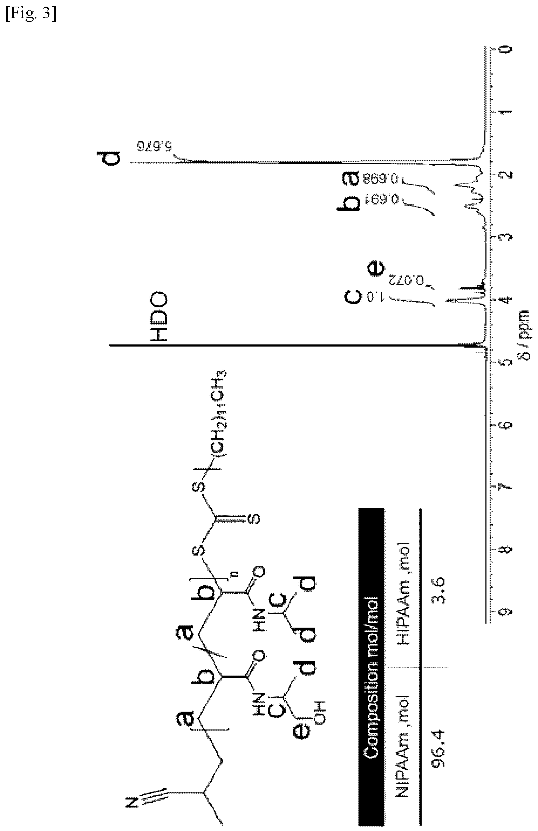

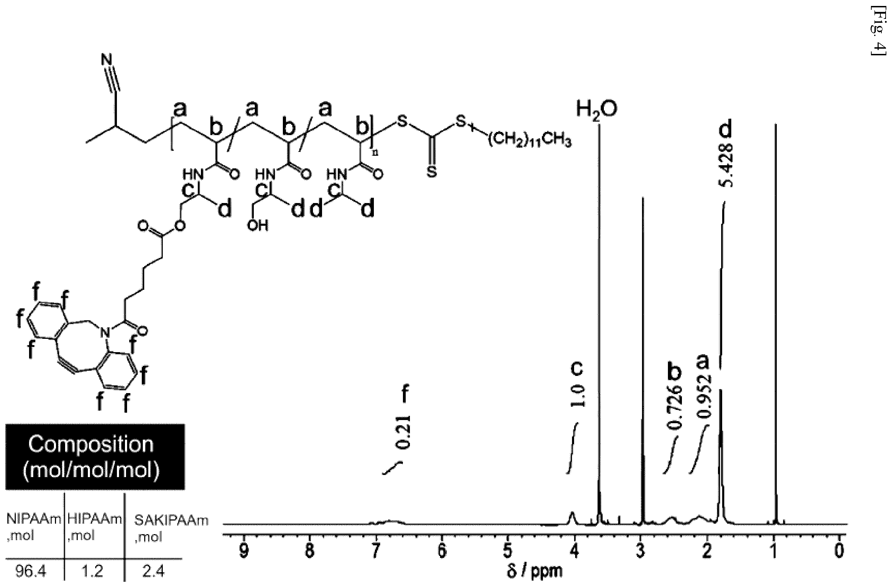

As the results of ¹H NMR analysis, it was confirmed that the LCST of P(NIPAAm), which is known to be about 32°C, was successfully increased up to 37.4°C by polymerization of HIPAAm and NIPAAm at a ratio of 3.6 : 96.4 (on a molar basis) and introduction of the hydroxyl group of HIPAAm and that a temperature responsive copolymer (I) was formed at a ratio of 96.4 : 1.2 : 2.4 (on a molar basis) by binding of an SAK group, which is a strained alkyne (Figs. 3 and 4).

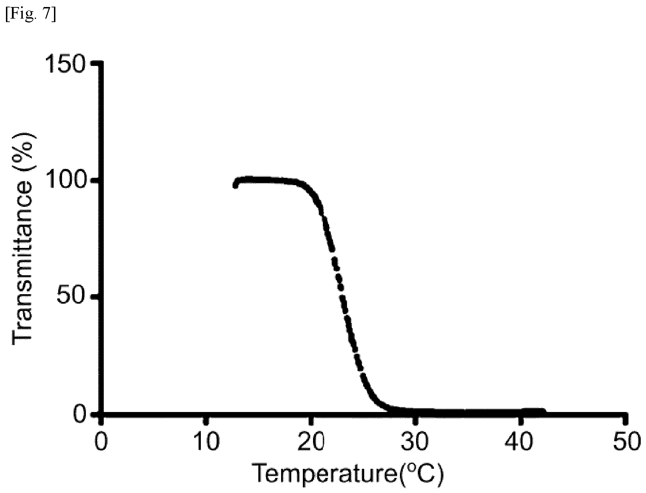

As shown in Figs. 3 to 7 and table 1, the results of GPC analysis demonstrated that the molecular weight of the synthesized polymer was changed from 1.904 × 10⁴ to 2.014 × 10⁴ by insertion of the SAK group.

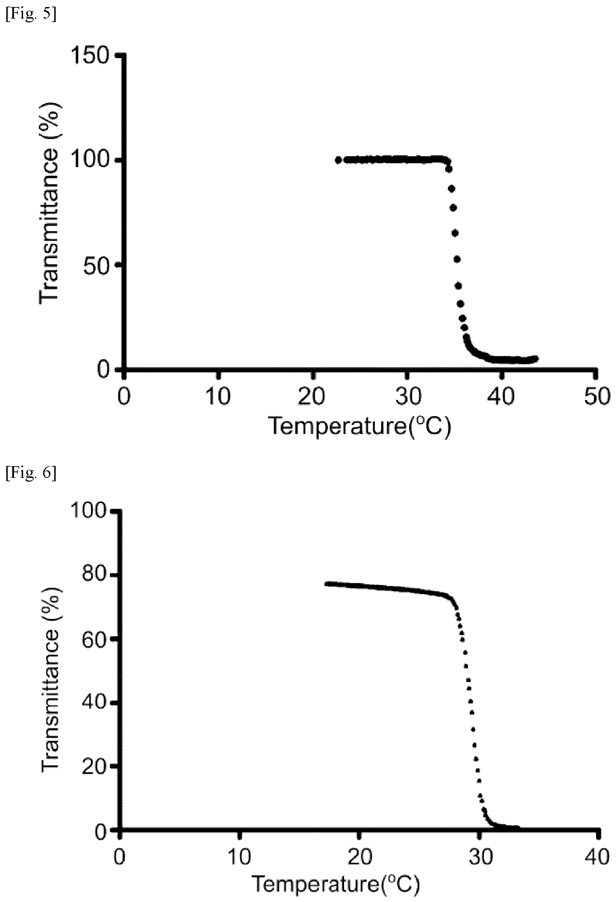

The results of LCST measurement of P(NIPAAm-co-HIPAAm), copolymer (I), and antibody-copolymer (I) conjugate are shown in Fig. 5, Fig. 6, and Fig. 7, respectively. The measurement was performed using PBS (pH 7.4) as the solvent, at a copolymer concentration of 2.0 mg/mL, a heating rate of 0.2°C/min, and a wavelength of 450 nm. The temperature at which the light transmittance was 50% was defined as the LCST. As a result, it was demonstrated that the LCST was changed from 37.4°C of P(NIPAAm-co-HIPAAm) to 30.1°C of copolymer (I).

**[Table 1]**

| Table 1 | Ratio of repeating unit determined by 1H NMR (mol/mol/mol) | | | LCST [°C] | Molecular weight [× 10⁴] |
|---|---|---|---|---|---|
| | NIPAAm | HIPAAm | SAKIPAAm | | |
| P(NIPAAm-HIPAAm) | 96.4 | 3.6 | 0 | 37.4 | 1.904 |
| Copolymer (I) | 96.4 | 1.2 | 2.4 | 30.1 | 2.014 |

### (Synthesis of azido- COVID-19 antibody)

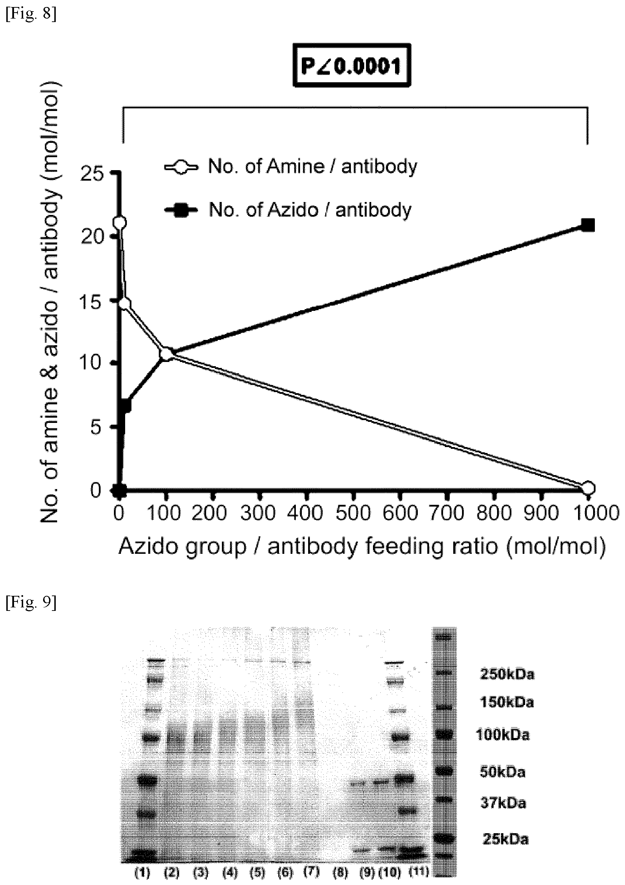

The azido group was introduced into the COVID-19 antibody by binding azido-(EG)₄-NHS to a lysine residue (ε-amino group) of the antibody. Confirmation and quantitative measurement of azido-(EG)₄-NHS was performed by a fluorescence reduction method. As shown in Fig. 8, the binding amount of the azido group gradually increased (0, 6.7, 10.7, and 20.9 mol/mol) by increasing the amount of azido-(EG)₄-NHS, and the binding amount of the amine group gradually decreased as follows (21.1, 14.7, 10.7, and 0.2 mol/mol). P-value < 0.0001.

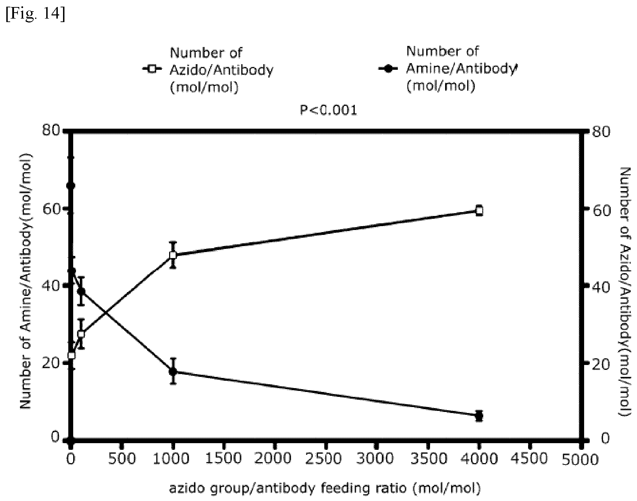

Synthesis of an azido- COVID-19 antibody was attempted as in above except that "SARS-CoV-2 Spike Protein (S1-NTD) Antibody #56996" (Cell Signaling Technology, Inc.) was used as the antibody instead of anti- COVID-19 monoclonal antibody (> 95%, manufactured by MyBioSource, Inc., anti-COVID-19 antibody :: anti-viral COVID 19 nucleocapsid (NP) humanized coronavirus monoclonal antibody), and the synthesis similarly succeeded. Fig. 14 shows the results. When the amount of azido-(EG)₄-NHS was increased, the binding amount of the azido group gradually increased, and the binding amount of the amine group gradually decreased.

### (Confirmation of the synthesized anti-COVID-19-copolymer (I) conjugate synthesized by click reaction)

Fig. 9 shows gel images of conjugates of COVID-19 antibody-copolymer (I) by sodium dodecylsulfate gel electrophoresis (SDS-PAGE).

Here, (1) and (11) are protein ladders, (2) to (7) are antibody : copolymer (I) at 1 : 1 (mol/mol), 1 : 2, 1 : 4, 1 : 8, 1 : 15, and 1 : 30, respectively, (8) is the copolymer (I) itself, (9) is azido-antibody, and (10) is antibody.

Investigation by changing the ratio of COVID-19 antibody and copolymer (I) to 1 : 1, 1 : 2, 1 : 4, 1 : 8, 1 : 15, and 1 : 30 (lanes 2 to 7) demonstrated that increasing the amount of polymer in the reaction solution caused more conjugation to the antibody and finally, more band broadening and increasing in the conjugate molecular weight.

These results indicate that a COVID-19 antibody-specific copolymer conjugate was successfully synthesized.

### (COVID-19 antibody-temperature-responsive polymer enrichment efficacy evaluation)

The COVID-19 antibody-temperature responsive copolymer showed highly significant concentration ability after thermal precipitation compared to a control (p = 0.0006).

### (Antigen enrichment using COVID-19 antibody-temperature responsive copolymer)

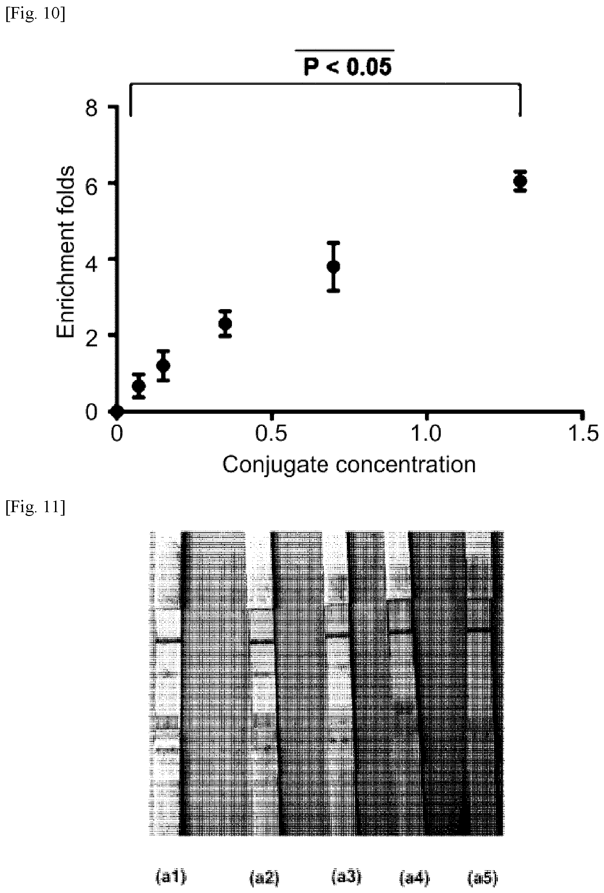

As shown in Fig. 10, the number of folds of the COVID-19 recombinant protein shifted from 0 to about six times the original antigen concentration at the highest conjugate concentration (1.3 mg/mL) by increasing the concentration of the COVID-19 antibody-copolymer conjugate, showing that the developed system has an ability of concentrating a sample containing a low amount of virus (p < 0.05). The unit of the horizontal axis of Fig. 10 is mg/mL.

### (Evaluation of antigen enrichment using lateral flow immunoassay strip)

As shown in Fig. 11, the minimum detectable limit of the COVID-19 recombinant protein using LFIA is about 50 pg/mL (a5), and although a weak band was observed in the test zone at 100 pg/mL (a4), as shown in (a1), (a2), and (a3) of Fig. 11, strong positive bands were observed at concentrations of 200 to 1000 pg/mL.

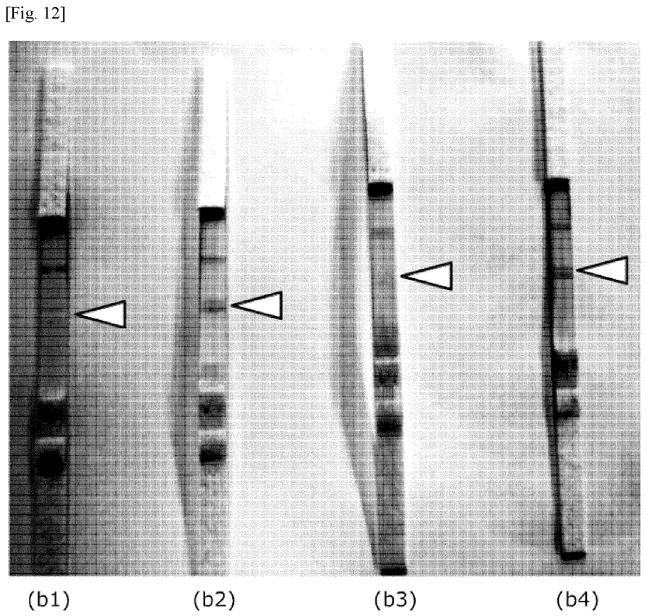

Fig. 12 shows comparison between before and after the enrichment: (b1) 50 pg/mL without enrichment, (b2) 50 pg/mL after enrichment, (b3) 100 pg/mL without enrichment, and (b4) 100 pg/mL after enrichment. It was demonstrated that strong positive bands (portions indicated by triangles in the figure) are observed by concentration using the COVID-19 antibody-copolymer conjugate.

### Reference Example [Synthesis 2 of P(NIPAAm-co-HIPAAm-co-SAKIPAAm)]

P(NIPAAm-co-HIPAAm-co-SAKIPAAm) was synthesized as in above except that in the synthesis of P(NIPAAm-co-HIPAAm-co-SAKIPAAm), the amount of NIPAAm to be added was changed from 1.89 g to 1.6 g and the amount of HIPAAm to be added was changed from 0.1 g to 0.0034 g. Hereinafter, the synthesized copolymer is referred to as "copolymer (II)".

The molecular weight of the copolymer (II) was 1.82 × 10⁴, and the introduction amount of SAKIPAAm was 14 mol% when the total of repeating units was defined as 100 mol%.

**[Table 2]**

| Table 2 | Ratio of repeating unit determined by 1H NMR (mol/mol/mol) | | | LCST [°C] | Molecular weight [× 10⁴] |
|---|---|---|---|---|---|
| | NIPAAm | HIPAAm | SAKIPAAm | | |
| P(NIPAAm-HIPAAm) | 77 | 23 | 0 | 35.3 | 1.72 |
| Copolymer (II) | 77 | 9 | 14 | 29.1 | 1.82 |

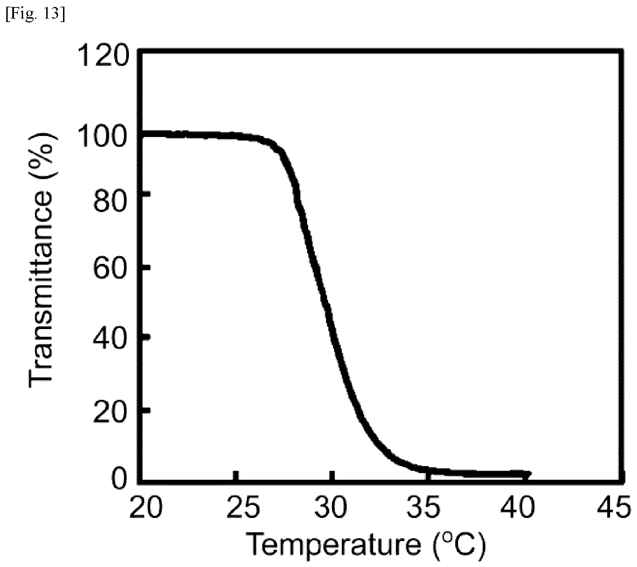

Fig. 13 shows the measurement results of LCST of synthesized copolymer (II). It was demonstrated from Fig. 13 that the LCST was 29.1°C.

The results above demonstrated that the copolymer (II) in which the content of SAKIPAAm unit (corresponding to the unit represented by the formula 4) having a click reaction site is from 5 mol% to 30 mol% or less when the total of repeating units is defined as 100 mol% can concentrate an antigen at lower temperature compared to the copolymer (I).

### Industrial Applicability

As described above, the copolymer can drastically reduce the false-positive rate that is caused due to a small amount of the object (such as antigen) in a specimen and can increase the accuracy of a test. In particular, it is possible to drastically increase the accuracy of a SARS-CoV-2 test by using the antibody-copolymer conjugate immobilizing a SARS-CoV-2 antibody.

## Claims

1. An antibody-copolymer conjugate comprising a repeating unit 1 represented by a following formula 1 and a repeating unit 7 represented by a following formula 7: 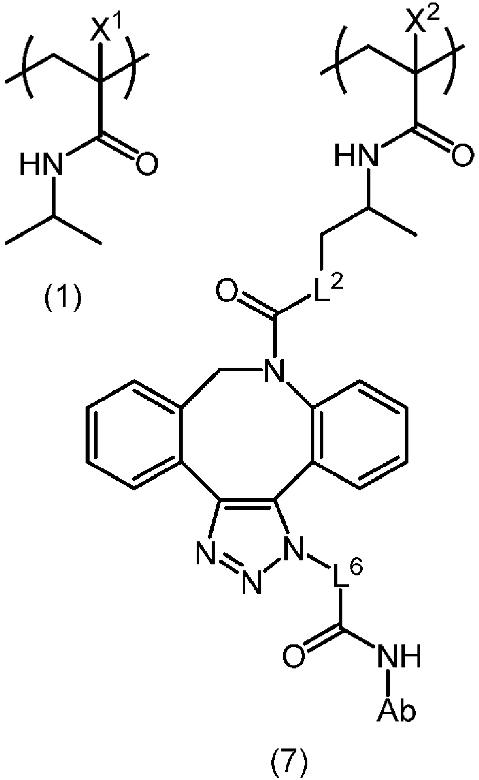 (in the formula 1, X¹ represents a hydrogen atom or a linear or branched alkyl group having 1 to 6 carbon atoms; and in the formula 7, X² represents a hydrogen atom or a linear or branched alkyl group having 1 to 6 carbon atoms, L² represents a divalent group, L⁶ represents a divalent hydrocarbon group that optionally includes a hetero atom, and Ab represents a residue of an antibody protein).

2. The antibody-copolymer conjugate according to Claim 1, wherein the Ab is an anti-SARS-CoV-2 antibody.

3. A method for concentrating an antigen comprising:
preparing a mixture solution A containing the antibody-copolymer conjugate according to Claim 1 or 2 and an antigen corresponding to the antibody and forming a complex of the antibody-copolymer conjugate and the antigen; and
heating the mixture solution A to 20°C to 40°C to precipitate the complex.

4. The method for concentrating an antigen according to Claim 3, further comprising:
binding a linker represented by a following formula 6 and the antibody via an amide bond derived from an amino group of the antibody to prepare an antibody-linker complex;
preparing a mixture solution B containing (i) the antibody-linker complex, and (ii) a copolymer comprising a repeating unit 1 represented by a following formula 1 and repeating unit 4 represented by a following formula 4; and
reacting and binding an azido group of the antibody-linker complex and an alkynylene group of the copolymer in the mixture solution B to generate the antibody-copolymer conjugate: 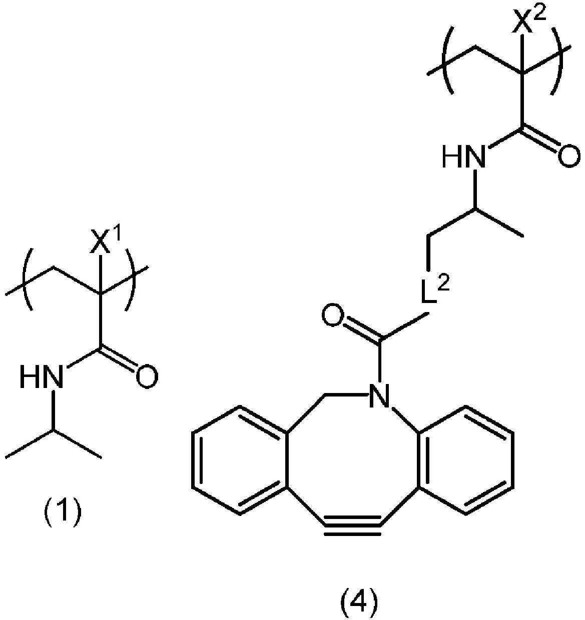 (in the formula 1, X¹ represents a hydrogen atom or a linear or branched alkyl group having 1 to 6 carbon atoms; and in the formula 4, X² represents a hydrogen atom or a linear or branched alkyl group having 1 to 6 carbon atoms, L² represents a divalent group), 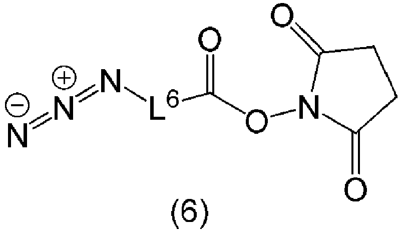 (in the formula 6, L⁶ represents a divalent hydrocarbon group that optionally includes a hetero atom).

5. The method for concentrating an antigen according to Claim 3 or 4, wherein the antibody is an anti-SARS-CoV-2 antibody.

6. The method for concentrating an antigen according to Claim 5, wherein the antigen is a SARS-CoV-2 protein.

7. The method for concentrating an antigen according to Claim 6, wherein a ratio of a content of the copolymer to a content of the antibody contained in the antibody-linker complex in the mixture solution B is 0.5 to 30 on a molar basis.

8. A method for detecting an antigen comprising concentrating the antigen by the method for concentrating an antigen according to any one of Claims 3 to 7 and detecting the concentrated antigen.

## Patentansprüche

1. Antikörper-Copolymer-Konjugat, umfassend eine Wiederholungseinheit 1, dargestellt durch die folgende Formel 1, und eine Wiederholungseinheit 7, dargestellt durch die folgende Formel 7: 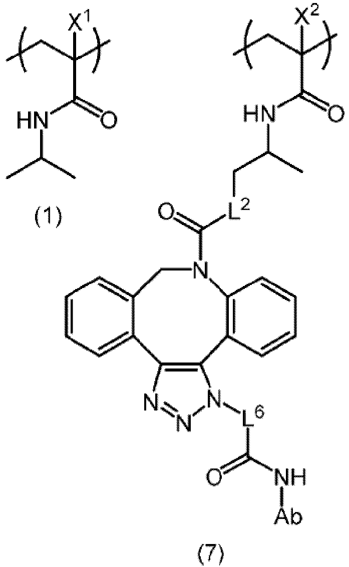 (in Formel 1 steht X¹ für ein Wasserstoffatom oder eine lineare oder verzweigte Alkylgruppe mit 1 bis 6 Kohlenstoffatomen; und in Formel 7 steht X² für ein Wasserstoffatom oder eine lineare oder verzweigte Alkylgruppe mit 1 bis 6 Kohlenstoffatomen, steht L² für eine zweiwertige Gruppe, steht L⁶ für eine zweiwertige Kohlenwasserstoffgruppe, die optional ein Heteroatom beinhaltet, und steht Ab für einen Rest eines Antikörperproteins).

2. Antikörper-Copolymer-Konjugat nach Anspruch 1, wobei der Ab ein Anti-SARS-CoV-2-Antikörper ist.

3. Verfahren zum Konzentrieren eines Antigens, umfassend:
Herstellen einer Mischungslösung A, die das Antikörper-Copolymer-Konjugat nach Anspruch 1 oder 2 und ein dem Antikörper entsprechendes Antigen enthält, und Ausbilden eines Komplexes aus dem Antikörper-Copolymer-Konjugat und dem Antigen; und
Erhitzen der Mischungslösung A auf 20 °C bis 40 °C, um den Komplex auszufällen.

4. Verfahren zum Konzentrieren eines Antigens nach Anspruch 3, ferner umfassend:
Binden eines Linkers, der durch die folgende Formel 6 dargestellt ist, und des Antikörpers über eine Amidbindung, die von einer Aminogruppe des Antikörpers abgeleitet ist, um einen Antikörper-Linker-Komplex herzustellen;
Herstellen einer Mischungslösung B, die (i) den Antikörper-Linker-Komplex und (ii) ein Copolymer enthält, das eine Wiederholungseinheit 1, die durch die folgende Formel 1 dargestellt ist, und eine Wiederholungseinheit 4, die durch die folgende Formel 4 dargestellt ist, umfasst; und
Reagieren und Binden einer Azidogruppe des Antikörper-Linker-Komplexes und einer Alkinylengruppe des Copolymers in der Mischungslösung B, um das Antikörper-Copolymer-Konjugat zu erzeugen: 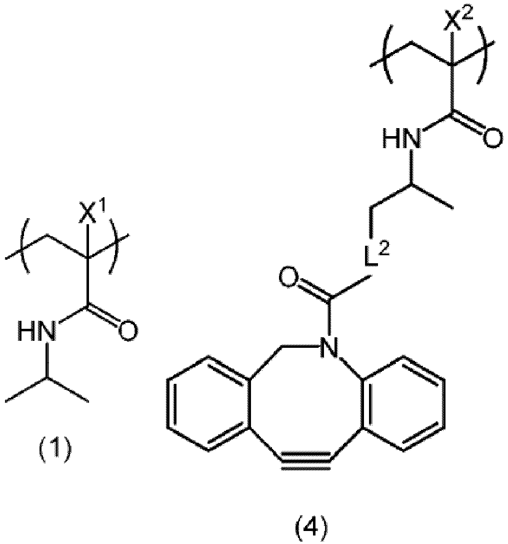 (in Formel 1 steht X¹ für ein Wasserstoffatom oder eine lineare oder verzweigte Alkylgruppe mit 1 bis 6 Kohlenstoffatomen; und in Formel 4 steht X² für ein Wasserstoffatom oder eine lineare oder verzweigte Alkylgruppe mit 1 bis 6 Kohlenstoffatomen, steht L² für eine zweiwertige Gruppe), 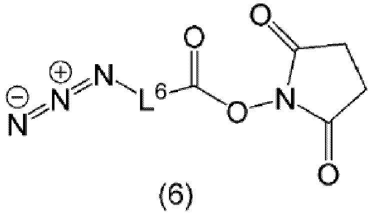 (in der Formel 6 steht L⁶ für eine zweiwertige Kohlenwasserstoffgruppe, die optional ein Heteroatom beinhaltet).

5. Verfahren zum Konzentrieren eines Antigens nach Anspruch 3 oder 4, wobei der Antikörper ein Anti-SARS-CoV-2-Antikörper ist.

6. Verfahren zum Konzentrieren eines Antigens nach Anspruch 5, wobei das Antigen ein SARS-CoV-2-Protein ist.

7. Verfahren zum Konzentrieren eines Antigens nach Anspruch 6, wobei ein Verhältnis des Gehalts des Copolymers zu dem Gehalt des in dem Antikörper-Linker-Komplex enthaltenen Antikörpers in der Mischungslösung B auf molarer Basis 0,5 bis 30 beträgt.

8. Verfahren zum Nachweisen eines Antigens, umfassend das Konzentrieren des Antigens durch das Verfahren zum Konzentrieren eines Antigens nach einem der Ansprüche 3 bis 7 und das Nachweisen des konzentrierten Antigens.

## Revendications

1. Conjugué anticorps-copolymère comprenant une unité de répétition 1 représentée par la formule 1 suivante et une unité de répétition 7 représentée par la formule 7 suivante : 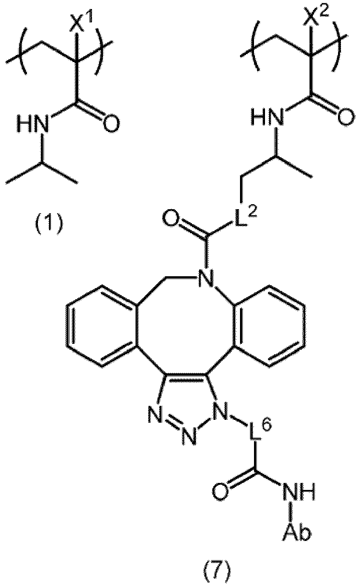 (dans la formule 1, X¹ représente un atome d'hydrogène ou un groupe alkyle linéaire ou ramifié ayant 1 à 6 atomes de carbone, et dans la formule 7, X² représente un atome d'hydrogène ou un groupe alkyle linéaire ou ramifié ayant 1 à 6 atomes de carbone, L² représente un groupe divalent, L⁶ représente un groupe hydrocarboné divalent qui comprend éventuellement un hétéroatome et Ab représente un résidu d'une protéine d'anticorps).

2. Conjugué anticorps-copolymère selon la revendication 1, dans lequel Ab est un anticorps anti-SARS-CoV-2.

3. Procédé de concentration d'un antigène comprenant :
la préparation d'une solution mixte A contenant le conjugué anticorps-copolymère selon la revendication 1 ou 2 et un antigène correspondant à l'anticorps, et la formation d'un complexe du conjugué anticorps-copolymère et de l'antigène, et
le chauffage de la solution mixte A de 20 °C à 40 °C pour précipiter le complexe.

4. Procédé de concentration d'un antigène selon la revendication 3, comprenant en outre :
la liaison d'un liant représenté par la formule 6 suivante et de l'anticorps par le biais d'une liaison amide dérivée d'un groupe amino de l'anticorps pour préparer un complexe anticorps-liant,
la préparation d'un mélange de solution B contenant (i) le complexe anticorps-liant et (ii) un copolymère comprenant une unité de répétition 1 représentée par la formule 1 suivante et une unité de répétition 4 représentée par la formule 4 suivante, et
la réaction et la liaison d'un groupe azido du complexe anticorps-liant et d'un groupe alkynylène du copolymère dans la solution mixte B pour générer le conjugué anticorps-copolymère : 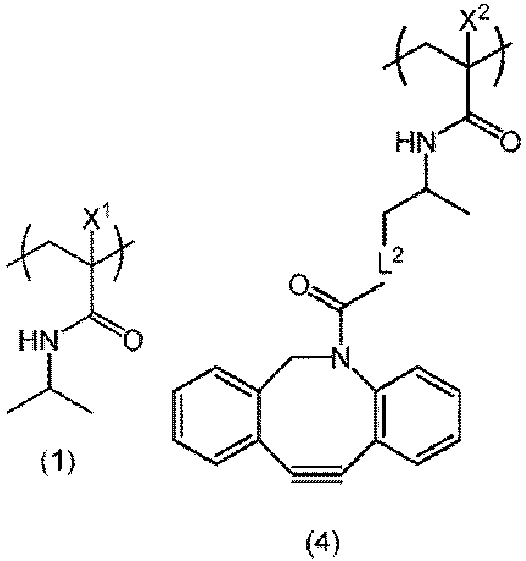 (dans la formule 1, X¹ représente un atome d'hydrogène ou un groupe alkyle linéaire ou ramifié ayant 1 à 6 atomes de carbone, et dans la formule 4, X² représente un atome d'hydrogène ou un groupe alkyle linéaire ou ramifié ayant 1 à 6 atomes de carbone, L² représente un groupe divalent), 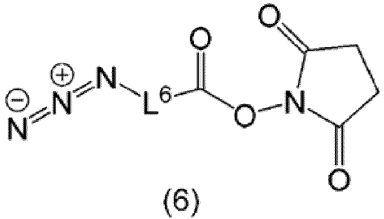 (dans la formule 6, L⁶ représente un groupe hydrocarboné divalent qui comprend éventuellement un hétéroatome).

5. Procédé de concentration d'un antigène selon la revendication 3 ou 4, dans lequel l'anticorps est un anticorps anti-SARS-CoV-2.

6. Procédé de concentration d'un antigène selon la revendication 5, dans lequel l'antigène est une protéine SARS-CoV-2.

7. Procédé de concentration d'un antigène selon la revendication 6, dans lequel le rapport de la teneur en copolymère à la teneur en anticorps contenu dans le complexe anticorps-liant dans la solution mixte B va de 0,5 à 30 sur une base molaire.

8. Procédé de détection d'un antigène comprenant la concentration de l'antigène au moyen du procédé de concentration d'un antigène selon l'une quelconque des revendications 3 à 7 et la détection de l'antigène concentré.
